(19) Europäisches Patentamt / European Patent Office / Office européen des brevets

(11) **EP 4 056 983 A1**

(12) **EUROPEAN PATENT APPLICATION**
published in accordance with Art. 153(4) EPC

(43) Date of publication:
**14.09.2022 Bulletin 2022/37**

(21) Application number: **20885871.2**

(22) Date of filing: **03.11.2020**

(51) International Patent Classification (IPC):
*G01N 1/28* (2006.01)    *G01N 21/84* (2006.01)

(86) International application number:
**PCT/CN2020/126249**

(87) International publication number:
**WO 2021/088826 (14.05.2021 Gazette 2021/19)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **04.11.2019 CN 201911065583**

(71) Applicants:
• **Shanghai King-Cell Biotechnology Co. Ltd.**
  **Shanghai 201507 (CN)**
• **Beijing Cell-Fusion Biotechnology Co., Ltd.**
  **Beijing 100085 (CN)**

(72) Inventors:
• **TIAN, Dayong**
  **Shanghai 201507 (CN)**
• **RUAN, Juncheng**
  **Shanghai 201507 (CN)**
• **FU, Zhenfang**
  **Shanghai 201507 (CN)**
• **ZHANG, Yajing**
  **Shanghai 201507 (CN)**
• **GU, Yulin**
  **Shanghai 201507 (CN)**
• **AN, Qi**
  **Shanghai 201507 (CN)**

(74) Representative: **Lavoix**
  **Bayerstraße 83**
  **80335 München (DE)**

(54) **DEVICE FOR EVALUATING NEUROVIRULENCE OF MUMPS VIRUS**

(57)    A device for evaluating the neurovirulence of a mumps virus, comprising: (I) a virus inoculation module, which is used for performing virus inoculation of a mumps virus to be evaluated on the lateral ventricle of a rat; (II) a processing module, which is used for performing vibration slicing on the fixed rat brain; (III) an imaging module, which is used for scanning and imaging the obtained rat brain slices; and (IV) an analysis module, which is used in the obtained imaging for calculating a neurovirulence index by using a formula I: the neurovirulence index = $S1/S0 \times 100$ (formula I) according to the cross-sectional area $S1$ of a cavity formed by hydrocephalus in the longitudinal section of the rat brain and the total cross-sectional area $S0$ of the rat brain. Multiple results show that the results are stable, repeatability is high, and a wild strain may be distinguished from a vaccine strain. In addition, relative to a current monkey body neurovirulence model, animal cost and difficulty of operation are greatly reduced.

Fig.4

**Description**

**TECHNICAL FIELD**

The present invention relates to the technical field of animal model construction, in particular to a rat model for evaluating neurovirulence of mumps virus.

**BACKGROUND**

Mumps virus is an RNA virus, belonging to the family Paramyxoviridae, which can spread through droplets and invade children's parotid gland and other glandular organs such as testes, ovaries, pancreas, kidneys and central nervous system. The clinical manifestations of the patient are enlargement of one or both parotid glands, accompanied by fever, fatigue, muscle pain, etc., with a certain probability of orchitis and encephalitis complications. At present, the mumps vaccine strains of the commercially available products are all attenuated strains obtained from the isolated wild virus strains through passage and weakening.

In view of the neurotoxicity of mumps virus, the evaluation of the neurovirulence of mumps vaccine is an important indicator of vaccine safety. At present, the method for evaluating the neurovirulence of mumps virus prescribed by most countries is monkey neurovirulence test. However, the monkey neurovirulence test requires a large number of non-human primates, which is difficult to operate and very expensive. More importantly, recent studies have shown that the monkey neurovirulence test is susceptible to various factors when detecting mumps virus strains, and the results may be unstable. Internationally, organizations such as the World Health Organization (WHO), the Food and Drug Administration (FDA) and the European Union (European Union, EU) have also questioned the scientificity of using monkeys as neurovirulence evaluation models for mumps virus and more objective animal models are being actively established.

In view of the above problems, it is urgent to establish an alternative animal model. STEVEN A. RUBIN et al. established a rat neurovirulence model for the first time. The specific method is to inoculate mumps virus into the lateral ventricle of Lewis suckling rats on the 1st day, and then the rats are euthanized on the 30th day. The rat brains are taken for paraffin sections and stained with hematoxylin-eosin (HE). The neurovirulence of mumps virus is evaluated by calculating the average percentage of the cross-sectional area of the cavities formed by hydrocephalus in the longitudinal sections of the rat brains in the brain (including the brainstem). Repeated experiments in multiple laboratories have shown that the rat neurovirulence model can distinguish between attenuated strains and wild strains, and is easy to operate with low cost. It is expected to gradually replace the monkey neurovirulence test in the future.

However, in the above-mentioned rat neurovirulence model, the slice processing takes a long time and the operation is complicated. More importantly, the processes of HE staining including multiple dehydration, transparency, and washing change the natural shape of brain slices, resulting in changes in brain cross-section, thereby resulting in artificial interference to the statistical analysis of the results. The current slice processing method is still unsatisfactory.

Therefore, there is an urgent need to develop a rat model for evaluating the neurovirulence of mumps virus with stable results, convenient operation and objective evaluation.

**SUMMARY OF THE INVENTION**

The purpose of the present invention is to provide a rat model for evaluating the neurovirulence of mumps virus rats with stable results, convenient operation and objective evaluation.

In the present invention, it provides a device for evaluating the neurovirulence of mumps virus, which comprises:

(I) a virus inoculation module for inoculating virus in the lateral ventricle of a rat with the mumps virus to be evaluated, and the rat is taken as sample i on the 20th to 30th days (preferably 22nd-28th days, more preferably 25th day) after virus inoculation;
(II) a processing module for taking the mouse brain from the sample i and fixing it, and performing vibration slicing on the fixed rat brain;
(III) an imaging module for scanning and imaging the rat brain slices obtained in the biological tissue slice module;
(IV) an analysis module for counting the cross-sectional area S1 of the cavity formed by hydrocephalus in the longitudinal section of the rat brain and the total cross-sectional area S0 of the rat brain without the cerebellum in the imaging obtained by the imaging module, and calculating the neurovirulence index of the rat by Formula I.

$$\text{Neurovirulence index} = \text{cross-sectional area S1 of cavity} / \text{total cross-sectional area S0 of brain} \times 100 \quad (\text{Formula I})$$

In another preferred embodiment, the rat is selected from the group consisting of a Wistar rat or a Lewis rat.

In another preferred embodiment, the rat is 1 to 3 days old, preferably 1 to 2 days old, and more preferably 1 day old.

In another preferred embodiment, the virus inoculation module comprises a syringe, which is a 20 $\mu$L micro-syringe with a size of 27G.

In another preferred embodiment, in the virus inoculation module, the position of the virus inoculation is between the bregrna and the lambda in the rat brain, 1.5 to 2.5 mm (preferably 1.9 to 2.1 mm, more preferably 2 mm) on the left side of the sagittal suture, and 1.5 to 2.5 mm (preferably 1.9 to 2.1 mm, more preferably 2 mm) in depth.

In another preferred embodiment, in the virus inoculation module, the volume of virus injected in the virus inoculation is 5 to 20 $\mu$L, preferably 8 to 15 $\mu$L, more preferably 10 $\mu$L.

In another preferred embodiment, in the virus inoculation module, the virus inoculation amount is $10^2$ to $10^4$ $CCID_{50}$, preferably $10^2$ to $10^3$ $CCID_{50}$, more preferably $10^2$ $CCID_{50}$.

In another preferred example, in the processing module, the following elements are included:

(a) an anesthesia element for anesthetizing sample i by injecting an anesthetic into sample i to obtain sample ii;
(b) an optional cardiac perfusion element for cardiac perfusion of sample ii to obtain sample iii;
(c) a fixing element for fixing the rat brain of sample iii;
(d) embedding and slicing elements.

In another preferred embodiment, the anesthetic element comprises an anesthetic and an anesthetic syringe.

In another preferred embodiment, the anesthetic is selected from the group consisting of chloral hydrate, pentobarbital sodium, urethane, and a combination thereof.

In another preferred embodiment, the anesthetic agent is chloral hydrate, and the concentration of chloral hydrate is 5% to 15%, preferably 10%.

In another preferred embodiment, the anesthetic is injected by intraperitoneal injection or intramuscular injection.

In another preferred embodiment, the injection amount of the anesthetic is 0.2 to 3 mL, preferably 0.5 to 1.5 mL, more preferably 1 mL.

In another preferred embodiment, the fixing element comprises a fixing solution selected from the group consisting of paraformaldehyde, acetone, ethanol, and a combination thereof.

In another preferred embodiment, the fixing solution is polyoxymethylene, and the concentration of the polyoxymethylene is 3% to 5%, preferably 4%.

In another preferred embodiment, the fixing time is 12 to 72 hours, preferably 20 to 28 hours, more preferably 24 hours.

In another preferred embodiment, an embedding material is included in the embedding and slicing element.

In another preferred embodiment, the embedding material is selected from the group consisting of agarose, paraffin wax, gelatin, and a combination thereof.

In another preferred embodiment, in the embedding and slicing element, vibration slicing can be performed.

In another preferred embodiment, in the analysis module, the statistical longitudinal section of the rat brain includes an area of 1 to 3 mm on the left side of the sagittal suture of the rat's left brain, preferably an area of 1.5 to 2.5 mm.

In another preferred embodiment, the analysis module comprises a photo analysis software.

In another preferred embodiment, the photo analysis software is selected from the group consisting of Image-pro plus or Image J.

The purpose of the present invention is to establish a rat model for evaluating the neurovirulence of mumps virus, replace the current monkey neurovirulence test, and provide a simple, low-cost and stable method for the safety evaluation of mumps vaccine.

To achieve this purpose, the method for establishing a rat neurovirulence model of the present invention comprises the following steps:

(1) The lateral ventricle of suckling rat is inoculated with mumps virus in a volume of 10 $\mu$L, and the virus amount is 100 $CCID_{50}$ mumps virus.
(2) On the 25th day, the rat is anesthetized and perfused to the heart, and the rat brain is taken out and fixed in 4% paraformaldehyde for 24 hours.
(3) The fixed rat brain is embedded with agarose and then vibration slicing is performed, and the rat brain slices are scanned and imaged.

(4) The average percentage of the cross-sectional area of the cavity formed by hydrocephalus in the longitudinal section of the rat brain to the total cross-sectional area of the brain is counted and designated as the rat neurovirulence index.

In a preferred embodiment, the present invention provides a method for establishing a rat model for the evaluating the neurovirulence of mumps virus, wherein the rat is a Wistar rat.

In a preferred embodiment, the present invention provides a method for establishing a rat model for evaluating the neurovirulence of mumps virus, wherein in step (1), the suckling rat is 1 day old.

In a preferred embodiment, the present invention provides a method for establishing a rat model for evaluating the neurovirulence of the mumps virus, wherein the specific position of the lateral ventricle injection in step (1) is between the bregrna and the lambda in the rat left brain, 2 mm on the left side of the sagittal suture, and 2 mm in depth.

In a preferred embodiment, the present invention provides a method for establishing a rat model for evaluating the neurovirulence of mumps virus, wherein the injected virus volume in step (1) is 10 $\mu$L, the amount of virus is 100 $CCID_{50}$, the syringe used is a 20 $\mu$L micro-syringe with a size of 27G.

In a preferred embodiment, the present invention provides a method for establishing a rat model for evaluating the neurovirulence of mumps virus, wherein the anesthetic used in step (2) is 10% chloral hydrate, and the anesthetic is injected intraperitoneally with a volume of 1 mL.

In a preferred embodiment, the present invention provides a method for establishing a rat model for evaluating the neurovirulence of mumps virus, wherein the slicing method used in step (3) is vibration slicing, and the parameters are as follows: the forward speed of the blade is 1.50mm/s, the amplitude is 0.75mm, and the thickness of the rat brain slice is 80 $\mu$m.

In a preferred embodiment, the present invention provides a method for establishing a rat model for evaluating the neurovirulence of mumps virus, wherein the rat brain slices used for scanning and analysis in step (3) should be located in the area which is 2mm on the left side of the left brain sagittal suture.

In a preferred embodiment, the present invention provides a method for establishing a rat model for evaluating the neurovirulence of mumps virus, wherein the brain slice photo analysis software in step (4) is image pro plus, and the statistical method is cavity area of ventricular area in brain slice/total cross-sectional area of brain slice.

It should be understood that within the scope of the present invention, each technical features of the present invention described above and in the following (as examples) may be combined with each other to form a new or preferred technical solution, which is not listed here due to space limitations.

## DESCRIPTION OF THE DRAWINGS

Fig.1 shows a schematic diagram of the position of virus inoculation in Example 1.
Fig.2 shows a schematic diagram of the rat brain cavity in Example 1.
Fig.3 shows a schematic diagram of a rat brain slice in Example 1.
Fig.4 shows a diagram of the statistical method in Example 1.
Fig.5 shows a diagram of the statistical results in Example 1.
Fig.6 shows a schematic diagram of the effects of two slicing methods in the comparative example.

## DETAILED DESCRIPTION OF THE PREFERRED

After extensive and in-depth research and a large number of screening, the inventors have developed a rat model for evaluating the neurovirulence of mumps virus rats with stable results, convenient operation and objective evaluation for the first time. The experimental results show that in the construction of a rat model for evaluating the neurovirulence of mumps virus, the processing step of vibration section is used to replace the step of paraffin section-HE staining in the traditional method, and the direct analysis after the section can eliminate the HE staining step, which shortens the processing time by 2 to 3 days, and significantly reduces the difficulty of operation. In addition, in the model of the present invention, the use of Wistar rats instead of Lewis rats also solves the problem that Lewis rats are scarce and difficult to obtain. On this basis, the present invention is completed.

**The main advantages of the present invention include:**

1) Strong repeatability. Multiple results show that the neurovirulence model can distinguish wild strains, partially attenuated strains and attenuated strains.

2) Objectivity and accuracy. The influence of artificial operations on the results during the section processing is excluded, which objectively reflects the cavity of brain slices.

3) Easy to operate. The rat brain can be directly statistically analyzed after slicing, and no other processing is required.

4) Low cost. Replacing monkeys with rats greatly reduces animal costs and feeding costs.

The present invention is further explained below in conjunction with specific example. It should be understood that these examples are only for illustrating the present invention and not intend to limit the scope of the present invention. The conditions of the experimental methods not specifically indicated in the following examples are usually in accordance with conventional conditions as described in Sambrook et al., Molecular Cloning: A Laboratory Manual (New York: Cold Spring Harbor Laboratory Press, 1989), or according to the conditions recommended by the manufacturer. Unless otherwise stated, percentages and parts are percentages by weight and parts by weight.

**Example 1: Establishment of the Rat Model for Evaluating Neurovirulence of Mumps Virus**

**1.1 Animal and Virus Dilution**

Four SPF Wistar pregnant rats were selected and randomly divided into 4 groups, namely A, B, C and D (the number of suckling rats in each group was not less than 10). Among them, group A was the control group inoculated with DMEM medium, group B was inoculated with vaccine strains, group C was inoculated with wild mumps virus strains passaged by Vero cells for 10 times, and group D was inoculated with wild mumps virus strains. In groups B, C and D, the virus titer was diluted with DMEM medium to 4.0 lg CCIDso/mL.

**1.2 Virus Inoculation**

After the mother rats gave birth, the 1-day-old suckling rats were fixed, and the sterilized 20 $\mu$L micro-syringe (size 27G) was used to absorb 10 $\mu$L virus solution. The injection position is between the bregma and the lambda in the rat left brain, 2 mm on the left side of the sagittal suture, and 2 mm in depth. Each virus was inoculated with 10 rats, and the group of blank control was injected with 10 $\mu$L DMEM medium in the same operation.

**1.3 Anesthesia and Cardiac Perfusion in Rat**

On the 25th day after virus inoculation, 1 mL 10% chloral hydrate solution was absorbed and injected intraperitoneally into rats. After 2 minutes, the rats were anesthetized and fixed in supine position, and the heart was perfused with 30 mL of PBS and 30 mL of 4% paraformaldehyde.

**1.4 Brain harvesting and fixation**

The skull was carefully removed and the rat brain was harvested as intact as possible, including the brain stem. The rat brains were immersed in 4% paraformaldehyde solution and placed in a refrigerator at 4°C for 24 hours.

**1.5 Embedding and Slicing of Rat Brain**

A certain amount of agarose was weighted and heated to dissolve in PBS for the preparation of 5% agarose gel. When the agarose solution is slightly cooled but not solidified, it was poured into a 6-well plate and the rat brain was placed horizontally. During the solidification of the agarose solution, adjust and maintain the horizontal posture of the rat brain.

After 2 hours, the rat brain that has been embedded in agarose gel was taken out of the 6-well plate, and the rat brain was cut in half vertically along the sagittal suture of the rat brain with a blade. The part containing the left hemisphere was cut into a cuboid glue block containing the intact left brain, and it was fixed on the microtome stage with glue. After the glue has solidified, PBS was poured into the carrier tank until overflowing the agarose gel.

Set the starting position of slicing and other parameters to start the slicing (the forward speed of the blade is set to 1.50 mm/s, the amplitude is set to 0.75mm, and the thickness of slice is set to 80 $\mu$m). An intact slice at a distance of 2 mL from the sagittal suture of the rat brain was selected, and the brain slice was removed from the PBS with a writing brush and attached to a glass slide. Maintain the natural shape of the slice and place the slide in the scanner for scanning and photographing.

**1.6 Statistical analysis**

The software image pro plus was used to analyze the slice photos of each group and calculate the neurovirulence index of each group.

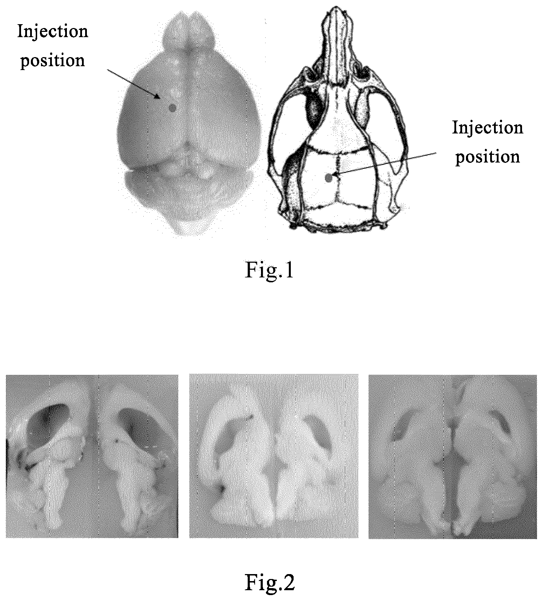

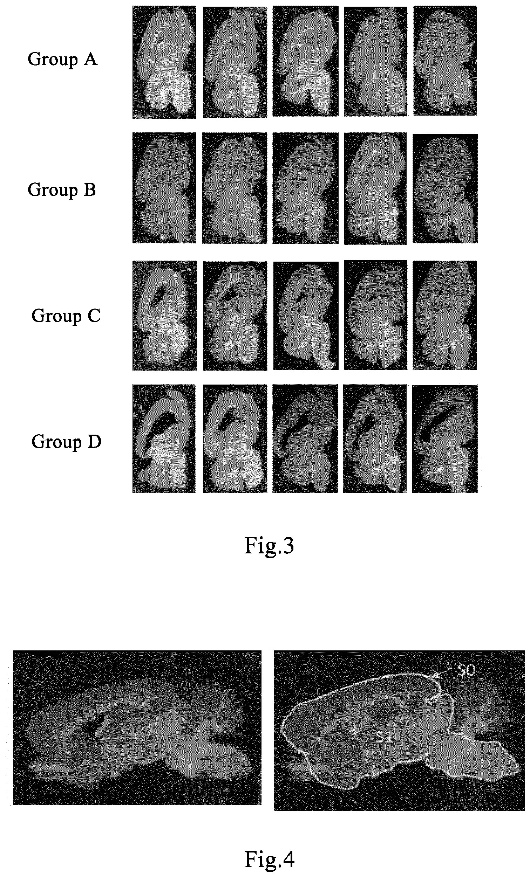

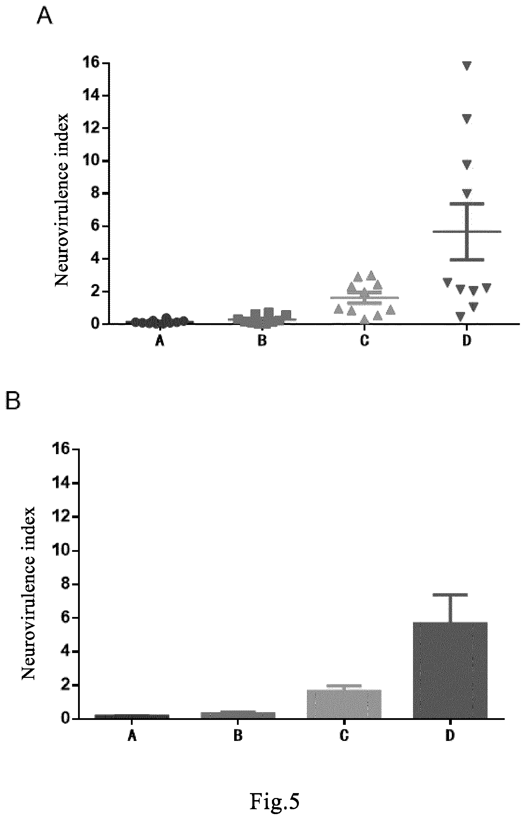

After the rat brains in group A and group B were fixed, some of the rat brains had obvious cavities after incision, as shown in Fig.2. After vibration slicing, some rat brain slices in each group are shown in Fig.3. The cavities in group A (DMEM group) and group B (vaccine strain group) are not obvious, while obvious cavities are found in some rat brains in group C (wild strain passage group) and group D (wild strain group). The final neurovirulence index statistical results are shown in Fig.5. The average score of group A is 0.15, the average score of group B is 0.31, the average score of group C is 1.63, and the average score of group D is 5.66. There is no significant difference between groups A and B, but there is significant difference between groups B, C and D (P<0.05).

**Comparative Example: Effect Test of the Rat Model for Evaluating the Neurovirulence of Mumps Virus-Comparison of Brain Tissue Processing Methods**

**2.1 Animals and Grouping**

Two SPF Wistar pregnant rats were divided into E and F groups. Group E was a group of the wild mumps virus strain, in which half of the rat brains were treated with vibration slicing, and the rest rat brains were treated with paraffin section-HE staining. Group F was a DMEM control group, and the postprocessing method after harvesting brain was consistent with that of group E.

**2.2 Virus Inoculation**

The titer of wild mumps virus strain was diluted to 4.0 lg CCIDso/mL. The 1-day-old suckling rat was fixed, and the sterilized 20 µL micro-syringe (size 27G) was used to absorb 10 µL virus solution to inoculate the left ventricle of the suckling rat. The inoculation position is between the bregrna and the lambda in the rat left brain, 2 mm on the left side of the sagittal suture, and 2 mm in depth. The group of blank control was injected with 10 µL DMEM medium in the same operation.

**2.3 Anesthesia and Cardiac Perfusion in Rat**

On the 25th day after virus inoculation, 1 mL 10% chloral hydrate solution was absorbed and injected intraperitoneally into rats. After 2 minutes, the rats were anesthetized and fixed in supine position, and the heart was perfused with 30 mL of PBS and 30 mL of 4% paraformaldehyde.

**2.4 Brain harvesting and fixation**

The skull was carefully removed and the rat brain was harvested as intact as possible, including the brain stem. The rat brains were immersed in 4% paraformaldehyde solution and placed in a refrigerator at 4°C for 24 hours.

**2.5 Paraffin sections and hematoxylin-eosin (HE) staining**

2.5.1 Dehydration and Transparency. A suitable size of brain tissue was cut and placed in a disposable plastic dehydration embedding box, and washed with running water for 1 hour. Ethanol gradient dehydration and xylene transparency were performed at room temperature (25°C), specifically, 50% ethanol for 2 hours →60% ethanol for 2 hours → 75% ethanol for 2 hours → 85% ethanol for 2 hours → 95% ethanol for 1 hour → absolute ethanol for 1 hour → xylene for 20 minutes. The result of transparency was observed. Suck up the liquid on the peripheral surface of the embedding box, and transfer it into the dipping wax.

2.5.2 Waxdip and embedding. The rat brain tissue block was immersed in paraffin solution at 60 °C for 2 hours, and then a certain wax solution is injected into the metal embedding mold, put the brain tissue with the section face downward to avoid generating bubbles.

2.5.3 Slicing and baking. The embedded tissue block was repaired and fixed, and the slice thickness was set to 8 µm for slicing. The wax slices with intact section were flattened in warm water at 42 °C with a writing brush, and laid flat on the glass slide. The wax slices were baked at 60 °C for 2 hours and then stored in a slicing box in order.

2.5.4 Hematoxylin-eosin (HE) staining. The slices were put into xylene for 40 minutes, absolute ethanol for 10 minutes, 75% alcohol for 5 minutes, and washed with tap water. The slices were put into hematoxylin staining solution for 3-5 minutes and washed with tap water. The slices were successively dehydrated with gradient alcohol of 85% and 95% for 5 minutes respectively, and stained with eosin staining solution for 5 minutes. The slices were successively put into anhydrous ethanol for 15 minutes, xylene for 10 minutes to transparent, and then sealed with neutral gum.

**2.6 Vibration slicing**

2.6.1 Rat brain embedding

A certain amount of agarose was weighted and heated to dissolve in PBS for the preparation of 5% agarose gel. When the agarose solution is slightly cooled but not solidified, it was poured into a 6-well plate and the rat brain was placed horizontally. During the solidification of the agarose solution, adjust and maintain the horizontal posture of the rat brain.

2.6.2 Slicing

After 2 hours, the rat brain that has been embedded in agarose gel was taken out of the 6-well plate, and the rat brain was cut in half vertically along the sagittal suture of the rat brain with a blade. The part containing the left hemisphere was cut into a cuboid glue block containing the intact left brain, and it was fixed on the microtome stage with glue. After the glue has solidified, PBS was poured into the carrier tank until overflowing the agarose gel.

Set the starting position of slicing and other parameters to start the slicing (the forward speed of the blade is set to 1.50 mm/s, the amplitude is set to 0.75mm, and the thickness of slice is set to 80 $\mu$m). An intact slice at a distance of 2 mL from the sagittal suture of the rat brain was selected, and the brain slice was removed from the PBS with a writing brush and attached to a glass slide.

**2.7 Image acquisition and analysis**

After taking photos of the slices, the results of vibration slicing and paraffin section-HE staining in the E and F groups were compared and analyzed.

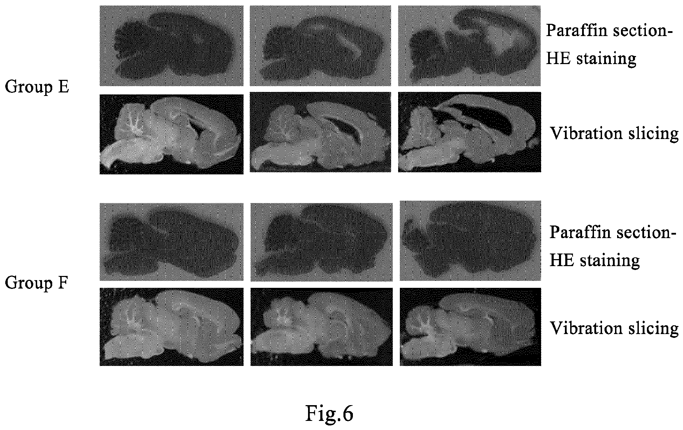

As shown in Fig.6, after vibration slicing treatment and paraffin section-HE staining treatment, in the wild virus strain group, cavities in the cross-section of brain can be seen, but there are no cavities in the DMEM group. There is no significant difference in the final statistical results between the two processing methods, but the paraffin section-HE staining processing cycle is long and the operation is complicated, which may easily lead to shrinkage, folding, and deformation of the slice, change the natural shape of the slice, and artificially change the cross-section of the brain, which interferes with the analysis.

All literatures mentioned in the present application are incorporated by reference herein, as though individually incorporated by reference. In addition, it should be understood that after reading the above teaching content of the present invention, various changes or modifications may be made by those skilled in the art, and these equivalents also fall within the scope as defined by the appended claims of the present application.

**Claims**

1. A device for evaluating the neurovirulence of mumps virus, which comprises:

    (I) a virus inoculation module for inoculating virus in the lateral ventricle of a rat with the mumps virus to be evaluated, and the rat is taken as sample i on the 20th to 30th days (preferably 22nd-28th days, more preferably 25th day) after virus inoculation;
    (II) a processing module for taking the mouse brain from the sample i and fixing it, and performing vibration slicing on the fixed rat brain;
    (III) an imaging module for scanning and imaging the rat brain slices obtained in the biological tissue slice module;
    (IV) an analysis module for counting the cross-sectional area S1 of the cavity formed by hydrocephalus in the longitudinal section of the rat brain and the total cross-sectional area S0 of the rat brain without the cerebellum in the imaging obtained by the imaging module, and calculating the neurovirulence index of the rat by Formula I.

$$\text{Neurovirulence index} = \text{cross-sectional area S1 of cavity} / \text{total cross-sectional area S0 of brain} \times 100 \text{ (Formula I)}$$

**2.** The device of claim 1, wherein the rat is selected from the group consisting of a Wistar rat or a Lewis rat.

**3.** The device of claim 1, wherein the rat is 1 to 3 days old, preferably 1 to 2 days old, and more preferably 1 day old.

**4.** The device of claim 1, wherein in the virus inoculation module, the position of the virus inoculation is between the bregrna and the lambda in the rat brain, 1.5 to 2.5 mm (preferably 1.9 to 2.1 mm, more preferably 2 mm) on the left side of the sagittal suture, and 1.5 to 2.5 mm (preferably 1.9 to 2.1 mm, more preferably 2 mm) in depth.

**5.** The device of claim 1, wherein in the virus inoculation module, the virus inoculation amount is $10^2$ to $10^4$ $CCID_{50}$, preferably $10^2$ to $10^3$ $CCID_{50}$, more preferably $10^2$ $CCID_{50}$.

**6.** The device of claim 1, wherein the processing module comprises the following elements:

(a) an anesthesia element for anesthetizing sample i by injecting an anesthetic into sample i to obtain sample ii;
(b) an optional cardiac perfusion element for cardiac perfusion of sample ii to obtain sample iii;
(c) a fixing element for fixing the rat brain of sample iii;
(d) embedding and slicing elements.

**7.** The device of claim 6, wherein the anesthetic element comprises an anesthetic and an anesthetic syringe.

**8.** The device of claim 7, wherein the anesthetic is injected by intraperitoneal injection or intramuscular injection.

**9.** The device of claim 6, wherein the fixing element comprises a fixing solution selected from the group consisting of paraformaldehyde, acetone, ethanol, and a combination thereof.

**10.** The device of claim 1, wherein in the analysis module, the statistical longitudinal section of the rat brain includes an area of 1 to 2.5 mm on the left side of the sagittal suture of the rat's left brain, preferably an area of 1.8 to 2.2 mm.

Fig.1

Fig.2

Group A

Group B

Group C

Group D

Fig.3

Fig.4

Fig.5

Group E — Paraffin section-HE staining, Vibration slicing

Group F — Paraffin section-HE staining, Vibration slicing

Fig.6

## INTERNATIONAL SEARCH REPORT

| International application No. |
| --- |
| **PCT/CN2020/126249** |

**A.    CLASSIFICATION OF SUBJECT MATTER**

G01N 1/28(2006.01)i;  G01N 21/84(2006.01)i

According to International Patent Classification (IPC) or to both national classification and IPC

**B.    FIELDS SEARCHED**

Minimum documentation searched (classification system followed by classification symbols)

G01N 1, G01N 21

Documentation searched other than minimum documentation to the extent that such documents are included in the fields searched

Electronic data base consulted during the international search (name of data base and, where practicable, search terms used)

CNKI, CNTXT, VEN, 腮腺炎病毒, 神经毒力, 神经毒性, 横截面积, neurovirulence, neurotoxicity, mumps virus, cross-sectional area

**C.    DOCUMENTS CONSIDERED TO BE RELEVANT**

| Category* | Citation of document, with indication, where appropriate, of the relevant passages | Relevant to claim No. |
| --- | --- | --- |
| PX | CN 110736654 A (SHANGHAI K-CELL BIOTECH CO., LTD. et al.) 31 January 2020 (2020-01-31) claims 1-10 | 1-10 |
| Y | STEVEN A. RUBIN et al.,. "Evaluation of a neonatal rat model for prediction of mumps virus neurovirulence in humans" *Journal of Virology*, Vol. 74, No. 11, 30 June 2000 (2000-06-30), pp. 5382-5384 | 1-10 |
| Y | STEVEN A. RUBIN et al.,. "The rat-based neurovirulence safety test for the assessment of mumps virus neurovirulence in humans: An international collaborative study" *JOURNAL OF INFECTIOUS DISEASES*, Vol. 191, 01 April 2005 (2005-04-01), pp. 1123-1128 | 1-10 |
| Y | CHRISTIAN J. SAUDER et al.,. "Changes in mumps virus neurovirulence phenotype associated with quasispecies heterogeneity" *Virology*, Vol. 350, 21 February 2006 (2006-02-21), pp. 48-57 | 1-10 |

☑ Further documents are listed in the continuation of Box C.          ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **30 December 2020** | **02 February 2021** |

| Name and mailing address of the ISA/CN | Authorized officer |
| --- | --- |
| **China National Intellectual Property Administration (ISA/CN)** **No. 6, Xitucheng Road, Jimenqiao, Haidian District, Beijing 100088** **China** | |
| Facsimile No. **(86-10)62019451** | Telephone No. |

Form PCT/ISA/210 (second sheet) (January 2015)

## INTERNATIONAL SEARCH REPORT

| International application No. |
| --- |
| **PCT/CN2020/126249** |

**C. DOCUMENTS CONSIDERED TO BE RELEVANT**

| Category* | Citation of document, with indication, where appropriate, of the relevant passages | Relevant to claim No. |
| --- | --- | --- |
| Y | CN 109030431 A (SUZHOU BRAIN SPACE INFORMATION RESEARCH INSTITUTE, HUAZHONG UNIVERSITY OF SCIENCE AND TECHNOLOGY) 18 December 2018 (2018-12-18)<br>  description, paragraphs 65-69 | 1-10 |
| A | CN 106226509 A (THE FOURTH MILITARY MEDICAL UNIVERSITY OF PLA) 14 December 2016 (2016-12-14)<br>  entire document | 1-10 |
| A | CN 106023291 A (HUAZHONG UNIVERSITY OF SCIENCE AND TECHNOLOGY) 12 October 2016 (2016-10-12)<br>  entire document | 1-10 |
| A | EP 0701127 A1 (JAPAN POLIOMYELITIS RES INST et al.) 13 March 1996 (1996-03-13)<br>  entire document | 1-10 |

Form PCT/ISA/210 (second sheet) (January 2015)

**INTERNATIONAL SEARCH REPORT**
Information on patent family members

International application No.

**PCT/CN2020/126249**

| Patent document cited in search report | | | Publication date (day/month/year) | Patent family member(s) | | | Publication date (day/month/year) |
|---|---|---|---|---|---|---|---|
| CN | 110736654 | A | 31 January 2020 | None | | | |
| CN | 109030431 | A | 18 December 2018 | None | | | |
| CN | 106226509 | A | 14 December 2016 | CN | 106226509 | B | 06 September 2019 |
| CN | 106023291 | A | 12 October 2016 | CN | 106023291 | B | 17 May 2019 |
| | | | | WO | 2017193700 | A1 | 16 November 2017 |
| EP | 0701127 | A1 | 13 March 1996 | WO | 9525954 | A1 | 28 September 1995 |
| | | | | EP | 0701127 | B1 | 23 May 2001 |
| | | | | US | 5986171 | A | 16 November 1999 |
| | | | | JP | 2813066 | B2 | 22 October 1998 |
| | | | | CA | 2163229 | C | 14 March 2000 |

Form PCT/ISA/210 (patent family annex) (January 2015)

**EP 4 056 983 A1**

## REFERENCES CITED IN THE DESCRIPTION

**Non-patent literature cited in the description**

- **SAMBROOK et al.** Molecular Cloning: A Laboratory Manual. Cold Spring Harbor Laboratory Press, 1989